# EUROPEAN PATENT APPLICATION

(11) **EP 3 034 617 A1**
(43) Date of publication of application: **22.06.2016**
(21) Application number: 14198207.4
(22) Date of filing: 16.12.2014
(51) Int. Cl.: C12Q 1/68, B01L 3/00, B01L 7/00

(54) **Method for the simultaneous identification of multiple same-genome fragments or multiple fragments from different genomes using a PCR-technique**

(71) Applicant: Isamat Riviere, Marcos, 08022 Barcelona (ES)
(72) Inventor: Isamat Riviere, Marcos, 08022 Barcelona (ES)
(74) Representative: Balder IP Law, S.L.

(57) **Abstract**

Method for the simultaneous identification of multiple same-genome fragments or multiple fragments from different genomes using a PCR-technique

Method for the simultaneous identification of multiple same-genome fragments or multiple fragments from different genomes (1) using a PCR-technique comprising the following steps:
a) Providing a linear or two-dimensional solid support (2) on which the specific oligonucleotide primers (3) are attached in a specific identifiable position;
b) Introducing said support (2) in a specific reaction vessel (4) containing a conventional PCR mix (5) including specific double stranded DNA fluorescent label molecule (F), said PCR mix (5) excluding the primers (3) which are attached to the solid support (2);
c) Wherein said support (2) is printed with both forward and reverse PCR primers (3) in the same position and where such primers are synthesized according to a Dual Primer Oligonucleotide design, for each target genome fragment.
d) Adding to the PCR mix nucleic acids derived from a biological fluid;
e) Thermocycling the vessel (4) for amplification of target sequences present in the biological fluid from the primers (3) attached to the solid support (2);
f) Measuring in real time in the process of thermocycling or at end point the fluorescence intensities originating from the solid support (2) within the reaction vessel taking into account the position within the solid support (2);

Thus allowing for a fast identification method of DNA fragments contained in biological specimens.

## Description

### TECHNICAL FIELD

The present invention relates to a method for the simultaneous identification of multiple same-genome fragments or multiple fragments from different genomes using a PCR-technique that allows for a fast identification method of DNA fragments contained in biological specimens. Specifically the present invention is a variation of a solid phase PCR, wherein primers are covalently linked to a solid-support surface that enables multiplexing unlimited targets.

### STATE OF THE ART

Many variations on PCR have been developed since the introduction of PCR methods in research and diagnosis from 1990 including variations such as multiplex-PCR by which several primer sets within a single PCR mix produce amplicons of varying sizes originating from different DNA sequences to enable targeting multiple genes at once, or solid-phase PCR whereby one primer in a set of primers is covalently linked to a solid support surface enabling localization of resulting amplicons on the solid support.

However both techniques are limited in the number of possible targets due to difficulty in optimizing and matching annealing temperatures for each primer set leading to low level multiplexation in either PCR method.

On the other hand, the following documents relate to different aspects of the present invention:
EP0640828 discloses an apparatus for monitoring multiple nucleic acid amplifications simultaneously. In order to provide real-time monitoring of the amplification product of multiple nucleic acid amplifications simultaneously the apparatus is characterized in that it comprises a thermal cycler including a heat conducting member having multiple recesses formed therein; and a sensor (arranged for detecting light emitted from said recesses, simultaneously. However, it is a complex apparatus since it has to manage a plurality of test tubes at once.
EP1614472B1 discloses a luminometer for automatic analyses.
EP2228454 discloses a single-tube multiplex assay, capable of simultaneously amplifying, detecting and quantifying multiple nucleic acid targets, using multiple hybridization probes, labelled with the same fluorescent reporter label, but each having a distinct melting temperature. However, hybridisation takes a long time and the method is not able to offer results in a short period of time.
EP2099930 discloses a method that carries out a hybridisation method using a solid support. None of the methods or devices disclosed in the above-mentioned documents allows for a fast and cheap detection of DNA fragments contained in biological specimens.

### DESCRIPTION OF THE INVENTION

To overcome the above-mentioned drawbacks, the present invention proposes a method for the simultaneous identification of multiple same-genome fragments or multiple fragments from different genomes using a PCR-technique comprising the following steps:
a) Providing a solid support, preferably a linear or two-dimensional support, on which the specific oligonucleotide primers are attached in a specific identifiable position;
b) Introducing said support in a specific reaction vessel containing a conventional PCR mix including specific double stranded DNA fluorescent label molecule, said PCR mix excluding the primers which are attached to the solid support;
c) Wherein said support is printed with both forward and reverse PCR primers in the same position and where such primers are synthesized according to a Dual Primer Oligonucleotide design, for each target genome fragment.
d) Adding to the PCR mix nucleic acids derived from a biological fluid;
e) Thermocycling the vessel for amplification of target sequences present in the biological fluid from the primers attached to the solid support;
f) Measuring in real time in the process of thermocycling or at end point the fluorescence intensities originating from the solid support within the reaction vessel taking into account the position within the solid support;

Thus allowing for a fast identification method of DNA fragments contained in biological specimens.

The use of Dual Priming Oligonucleotide primers in conjunction with linear or to dimensional arrays enables matching annealing temperatures between primer sets without a limitation to the number of primer sets included in the array on the solid support, hence allowing for the formation of amplicons on pre-established and identifiable positions on the arrays included in the PCR mix, where these positions are only amplified when target DNA is present in the biological specimen, leading to binding of specific double-stranded DNA label molecules that generate a luminescent and detectable signal that locate original position of the primer set on the array providing the means for direct and immediate extrapolation of position on array to DNA sequence identity.

Method according to claim 1, wherein said nucleic acids come from infectious agents such as bacteria, virus and other pathogens, or from any other species genome, in biological fluids or tissues.

Method according to any of the previous claims, wherein the specific reaction vessel is a test tube.

Method according to any of the previous claims, wherein the solid support is a polymer or crystal based capillary,rod or two dimensional structure fitting into a reaction vessel and is printed accurately following a linear array with as many spots or rings, containing both forward and reverse primers in the same spot or ring, as the desired number identifiable genome targets.

Method according to any of the previous claims, wherein the tube containing the printed solid support fits into a real-time PCR thermocycling device with a new assembly of functional components that enable detection and reading of fluorescence intensities derived directly from the solid support within the vessel.

Device for carrying out the steps d) and e) comprising:
- A housing for receiving a test tube;
- Means for thermocycling the housing;
- A fluorescent light exciter and detector;

Wherein the housing comprises a vertical opening, the fluorescent light detector being arranged in front of or next to said opening such that it enables real-time or end-point measuring during PCR process of fluorescent intensities originating from a solid support placed within the reaction vessel taking into account the position within the solid support.

### BRIEF DESCRIPTION OF THE DRAWINGS

For a better understanding of the aforementioned, drawings are attached wherein, schematically and only by way of non-limitative example, several practical embodiments are represented.
Fig. 1 is a schematic representation of the basic arrangement for carrying out the method according to the invention, that is, a linear or two dimensional support placed and fixed into a test tube.
Fig. 2 is a zoom view of the linear or two dimensional support used in the invention, wherein the dual primer oligonucleotides are attached.
Fig. 3 shows the main components for carrying out the method of the invention.
Fig.4 shows the attachment of a DNA strand to the reverse and forward PCR primers.

### DESCRIPTION OF A WAY OF CARRYING OUT THE INVENTION

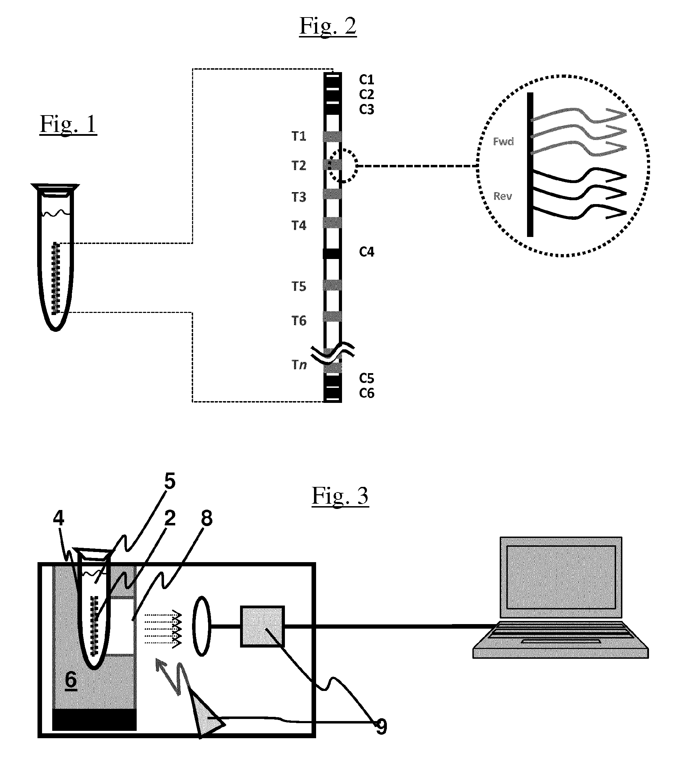

As shown in Figs. 1 to 5, the invention relates to a Method for the simultaneous identification of multiple same-genome fragments or multiple fragments from different genomes 1 using a PCR-technique comprising the following steps:
a) Providing a solid support 2, preferably a linear or two dimensional support, on which the specific oligonucleotide primers 3 are attached in a specific identifiable position;
b) Introducing said support 2 in a specific reaction vessel 4 containing a conventional PCR mix 5 including specific double stranded DNA fluorescent label molecule, said PCR mix 5 excluding the primers 3 which are attached to the solid support 2;
c) Wherein said support 2 is printed with both forward and reverse PCR primers 3 in the same position and where such primers are synthesized according to a Dual Primer Oligonucleotide design, for each target genome fragment.
d) Adding to the PCR mix nucleic acids derived from a biological fluid;
e) Thermocycling the vessel 4 for amplification of target sequences present in the biological fluid from the primers 3 attached to the solid support 2;
f) Measuring in real time in the process of thermocycling or at end point the fluorescence intensities originating from the solid support 2 within the reaction vessel taking into account the position within the solid support 2;

Thus allowing for a fast identification method of DNA fragments contained in biological specimens.

Said nucleic acids come from infectious agents such as bacteria, virus and other pathogens, or from any other species genome, in biological fluids or tissues.

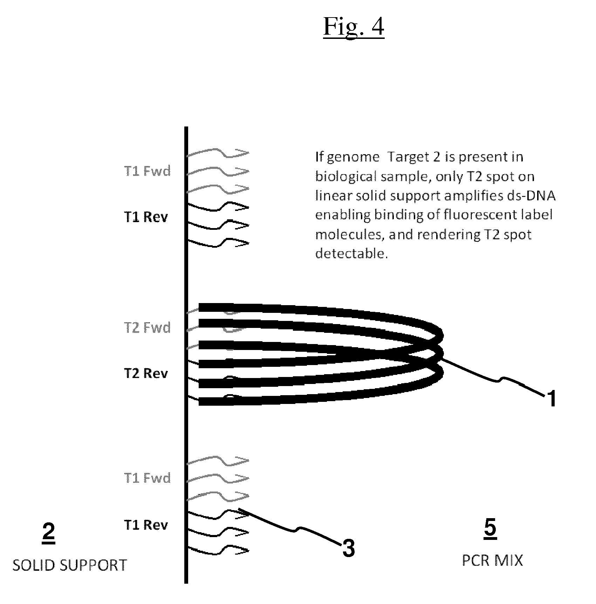

As shown in figure 4, the specific reaction vessel 4 is a test tube and the solid support is a polymer or crystal based capillary,rod or two dimensional structure fitting into a reaction vessel 4 and is printed accurately following a linear or 2D array with as many spots or rings, containing both forward and reverse primers in the same spot or ring, as the desired number identifiable genome targets.

The tube 4 containing the printed solid support 2 fits into a real-time PCR thermocycling device with a new assembly of functional components that enable detection and reading of fluorescence intensities derived directly from the solid support 2 within the vessel 4.

Fig. 4 shows a device for carrying out the steps d and e comprising:
- A housing 6 for receiving a test tube 4;
- Means for thermocycling the housing 6;
- A fluorescent light exciter and detector 7;

Wherein the housing 6 comprises a vertical opening 8, the fluorescent light detector 7 being arranged in front of said opening 8 such that it enables real-time or end-point measuring during PCR process of fluorescent intensities originating from a solid support 2 placed within the reaction vessel taking into account the position within the solid support 2.

A preferred application of the invention refers to clinical applications that are being developed in the field of periodontal disease and perimplantitis wherein a total of 14 periodontal anaerobic pathogen genome fragments and three human inflammatory gene variants serve as genetic diagnostic markers of subclinical disease which can be detected simultaneously by virtue of their placement on a linear array within the reaction vessel.

Other applications in a similar simultaneous detection approach refer specifically to cytokine, hormone and growth factor markers in the evaluation of the regenerative potential of stem cells, and feline or canine viral array vessels for detection of infectious agents in pet veterinary clinics or other infectious disease diagnosis in animal husbandry.

Applications for surface and/or environmental microbial sterility are also of straightforward implementation using this invention. Low genetic marker conditions amounting to less than 25 genome fragment targets, such as these, provide optimal simultaneous amplification targets by virtue of the low number complexity marker array required for distribution along a linear array to enable straightforward detection upon PCR amplification.

It would be obvious for a skilled person in the field to adapt this invention to cover conditions involving larger numbers of genetic markers to provide fast diagnostic tools to complex polygenic conditions such as human liver metabolism applied to oncological therapies or neuro-pharmacological drugs, by modifying the solid phase support adequately such as by the use of a 2D matrix and adapting the detection device or reader. The vessel and detection device in this invention could also be used sequentially by processing several tubes in order to maximize high throughput analytical or diagnostic capacity.

Although reference has been made to a specific embodiment of the invention, it is apparent to one skilled in the art that the described method is susceptible of numerous variations and modifications, and that all the details mentioned can be substituted by other technically equivalents without departing from the scope of protection defined by the appended claims.

## Claims

1. Method for the simultaneous identification of multiple same-genome fragments or multiple fragments from different genomes (1) using a PCR-technique comprising the following steps:
a) Providing a solid support (2) on which the specific oligonucleotide primers (3) are attached in a specific identifiable position;
b) Introducing said support (2) in a specific reaction vessel (4) containing a conventional PCR mix (5) including specific double stranded DNA fluorescent label molecule, said PCR mix (5) excluding the primers (3) which are attached to the solid support (2);
c) Wherein said support (2) is printed with both forward and reverse PCR primers (3) in the same position and where such primers are synthesized according to a Dual Primer Oligonucleotide design, for each target genome fragment.
d) Adding to the PCR mix nucleic acids derived from a biological fluid;
e) Thermocycling the vessel (4) for amplification of target sequences present in the biological fluid from the primers (3) attached to the solid support (2);
f) Measuring in real time in the process of thermocycling or at end point the fluorescence intensities originating from the solid support (2) within the reaction vessel taking into account the position within the solid support (2);
Thus allowing for a fast identification method of DNA fragments contained in biological specimens.

2. Method according to claim 1, wherein said support is a linear or 2D support (2).

3. Method according to any of the previous claims, wherein said nucleic acids come from infectious agents such as bacteria, virus and other pathogens, or from any other species genome, in biological fluids or tissues.

4. Method according to any of the previous claims, wherein the specific reaction vessel (4) is a test tube.

5. Method according to any of the previous claims, wherein the solid support is a polymer or crystal based capillary,rod or 2 dimensional structure fitting into a reaction vessel (4) and is printed accurately following a array with as many spots or rings, containing both forward and reverse primers in the same spot or ring, as the desired number identifiable genome targets.

6. Method according to any of the previous claims, wherein the tube (4) containing the printed solid support (2) fits into a real-time PCR thermocycling device with a new assembly of functional components that enable detection and reading of fluorescence intensities derived directly from the solid support (2) within the vessel (4).

7. Device for carrying out the steps d) and e) comprising:
- A housing (6) for receiving a test tube (4);
- Means for thermocycling the housing (6);
- A fluorescent light exciter and detector (7);
Wherein the housing (6) comprises a vertical opening (8), the fluorescent light detector (7) being arranged in front of said opening (8) such that it enables real-time or end-point measuring during PCR process of fluorescent intensities originating from a solid support (2) placed within the reaction vessel taking into account the position within the solid support (2).
